# EUROPÄISCHE PATENTSCHRIFT

(11) **EP 3 732 465 B1**
(45) Veröffentlichungstag und Bekanntmachung des Hinweises auf die Patenterteilung: **10.11.2021**
(21) Anmeldenummer: 18833897.4
(22) Anmeldetag: 28.12.2018
(51) Int. Cl.: G01N 21/17

(54) **MESSVORRICHTUNG ZUR ERMITTLUNG EINER MESSGRÖSSE EINES MESSGASES**
MEASURING DEVICE FOR ASCERTAINING A MEASURAND OF A MEASUREMENT GAS
PROCÉDÉ DE MESURE POUR DÉTERMINER UNE QUANTITÉ MESURÉE D'UN GAZ À MESURER

(30) Priorität: 28.12.2017 AT 510842017
(43) Veröffentlichungstag der Anmeldung: 04.11.2020
(73) Patentinhaber: AVL List GmbH, 8020 Graz (AT)
(72) Erfinder: REINGRUBER, Herbert, 8045 Graz (AT); SCHINDLER, Wolfgang, 8043 Graz (AT); HARMS, Klaus-Christoph, 8051 Thal/Graz (AT); REINISCH, Tristan, 8045 Graz (AT)
(74) Vertreter: Patentanwälte Pinter & Weiss OG
(86) Internationale Anmeldenummer: PCT/EP2018/097061
(87) Internationale Veröffentlichungsnummer: WO 2019/129834

(56) Entgegenhaltungen:
- AT-U2- 6 894
- US-A1- 2002 194 897
- BRAND C ET AL: "Pulsed-laser excitation of acoustic modes in open high-Q photoacoustic resonators for trace gas monitoring: results for C2H4", APPLIED OPTICS, OPTICAL SOCIETY OF AMERICA, WASHINGTON, DC; US, Bd. 34, Nr. 18, 20. Juni 1995 (1995-06-20) , Seiten 3257-3266, XP002486473, ISSN: 0003-6935, DOI: 10.1364/AO.34.003257

## Beschreibung

Die gegenständliche Erfindung betrifft eine Messvorrichtung zur Ermittlung einer Messgröße eines Messgases mittels eines photoakustischen Verfahrens, wobei die Messvorrichtung einen Strömungskanal für das Messgas mit zumindest einer Zuleitung, einer photoakustischen Messzelle und einer Ableitung aufweist.

Weiters betrifft die Erfindung ein Verfahren zur Unterdrückung von Schallstörungen in einer Messvorrichtung zur Ermittlung einer Messgröße eines Messgases mittels eines photoakustischen Verfahrens, wobei das Messgas über einen Strömungskanal geleitet wird, der über zumindest eine Zuleitung, eine photoakustische Messzelle und eine Ableitung verläuft.

Die photoakustische Spektroskopie (PAS) wird für Messverfahren in vielen Bereichen der Gas- und Partikelmesstechnik sehr erfolgreich eingesetzt. Dabei wird das zu untersuchende Messgas (Aerosol) mit Hilfe eines intensitätsmodulierten Lasers zu Dichte- bzw. Druckschwingungen in einer akustisch-resonanten Messzelle angeregt. Dieses Schallsignal wird von einem Mikrofon empfangen und in ein elektrisches Signal umgewandelt. Entsprechende Systeme werden beispielsweise zur Ermittlung der Massenkonzentration [mg/m³] von Rußpartikeln im Abgas von Verbrennungsmotoren eingesetzt.

Das vom Mikrofon aufgenommene akustische Frequenzspektrum beinhaltet auch unerwünschte Störfrequenzen aus unterschiedlichen Quellen zum Beispiel Strömungsgeräusch des einströmenden Messgases, Motorgeräusche oder auch verschieden Umgebungsgeräusche sowie Vibrationen (Körperschall). Dabei kann der Schall vor allem folgende Wege nehmen:
- Über die Zu/Ableitungen zur Messzelle als Luftschall bzw. als Körperschall, der von den Wandungen der Leitungen übermittelt wird
- Über die Umgebung der Messzelle ebenfalls als Luftschall oder auch als
   Körperschall, der zum Beispiel über die Befestigung der Messzelle übertragen wird Zur Störunterdrückung kann der Körperschall an der Messzellenbefestigung beispielsweise durch die Verwendung von Dämpfungselementen, wie etwa Gummipuffern, unterdrückt werden, das Gerätegehäuse kann mit doppelten Wandung versehen sein, um von der Umgebung stammenden Luftschall abzuschirmen, und potentielle geräteinterne Störquellen (Pumpen, Lüfter, usw.) können entsprechend gelagert werden (etwa über Gummipuffer) oder sie werden in einem externen Gehäuse verbaut. Weiters kann beispielsweise der Frequenzgang des Mikrofons und nachfolgender Verstärker entsprechend der Nutzfrequenz gewählt werden. Auch durch eine geeignete Materialauswahl können die Störungseinflüsse gezielt beeinflusst werden.

Trotz zahlreicher bereits umgesetzter Maßnahmen besteht weiterhin ein Bedarf an zusätzlichen Verbesserungen zur Störungsunterdrückung. Insbesondere Störungen, welche auf dem Luftschallweg über die Zu- bzw. Ableitung in die PAS-Messzelle gelangen, stellen ein großes Problem dar. Je höher der Entnahmefluss gewählt wird (was zur Verbesserung der Dynamik beiträgt), desto größer wird das Strömungsrauschen.

US 2002/194897 A1 offenbart ein photoakustisches Messinstrument zur Partikelmessung. Im Bereich des Einlasskanals der Messzelle sind ein Hochfrequenzfilter und ein Tieffrequenzfilter vorgesehen, welche Frequenzen oberhalb und unterhalb der Nutzfrequenz der Messzelle filtern. Der Tieffrequenzfilter ist als Helmholtzresonator ausgebildet.

Die Veröffentlichung BRAND C. et al.: "Pulsed-laser excitation of acoustic modes in open high-Q photoacoustic resonators for trace gas monitoring: results for C2H4", APPLIED OPTICS, OPTICAL SOCIETY OF AMERICA, WASHINGTON DC; US, Bd.34, Nr.18, 20. Juni 1995 offenbart eine photoakustische Messzelle, wobei im Einlasskanal und im Auslasskanal akustische Filter vorgesehen sind.

Die gegenständliche Erfindung hat die Aufgabe, Störgeräusche auch bei hohen Strömungsgeschwindigkeiten wirkungsvoll zu unterdrücken und die Messgenauigkeit und Zuverlässigkeit der Messvorrichtung zu erhöhen.

Diese und weitere Aufgaben werden durch eine Messvorrichtung der eingangs genannten Art erfindungsgemäß dadurch gelöst, dass die Messvorrichtung zumindest ein auf die Nutzfrequenz der Messzelle abgestimmtes akustisches Filterelement aufweist, das zumindest einen am Strömungskanal angeordneten Hohlraumresonator umfasst, welcher an der Zuleitung und/oder der Ableitung angeordnet ist und dass zumindest eines der akustischen Filterelemente auf eine negative Schalldruckverstärkung in einem ersten Frequenzbereich der die Grundfrequenz der Nutzfrequenz umfasst, ausgelegt ist. Vorzugsweise steht dabei das Innere des Hohlraumresonators in Verbindung mit dem Inneren des Strömungskanals. Das akustische Filterelement eliminiert effektiv Störfrequenzen im Bereich der Nutzfrequenz, wodurch das Störrauschen gezielt im Frequenzfeld verringert wird, in der die jeweilige Messzelle arbeitet. Als Hohlraumresonatoren sind einseitig zum Strömungskanal hin offene Hohlraumresonatoren bevorzugt. Die gewünschten Eigenschaften des akustischen Filters können über die Form, Größe und gegebenenfalls relative Anordnung einzelner Hohlraumresonatoren zueinander gezielt beeinflusst werden.

Als "Nutzfrequenz" wird im Zusammenhang mit der gegenständlichen Offenbarung die Frequenz bezeichnet, die zur Ermittlung der Messeigenschaft ausgewertet wird. Im Allgemeinen ist dies eine Resonanzfrequenz der Messzelle und im Allgemeinen stimmt die Nutzfrequenz mit der Anregefrequenz des Lasers der Messzelle überein. Gegebenenfalls kann eine Messzelle auch mit unterschiedlichen Nutzfrequenzen betrieben werden. In Messvorrichtungen zur Ermittlung der Massenkonzentration von Rußpartikeln im Abgas von Verbrennungsmotoren hat sich beispielsweise eine Nutzfrequenz im Bereich von etwa 1000 bis 12000 Hz als vorteilhaft erwiesen, wobei in Abhängigkeit von der jeweiligen Anwendung allerdings auch andere Frequenzbereiche sinnvoll genutzt werden können.

In vorteilhafter Weise ist die Achse des Hohlraumresonators normal auf die Achse des Strömungskanals ausgerichtet. Dies erlaubt eine einfache Konstruktion des Hohlraumresonators, dessen akustische Eigenschaften gut ermittelbar sind.

In vorteilhafter Weise ist zumindest ein Hohlraumresonator als beidseitiger Hohlraumresonator mit einem ersten und einem zweiten Halsabschnitt ausgebildet, wobei vorzugsweise der erste Halsabschnitt gegenüberliegend dem zweiten Halsabschnitt angeordnet ist. Dadurch lassen sich im Schalldruckverlauf die Wertspitzen der negativen Schalldruckverstärkung verbreitern.

In einer weiteren vorteilhaften Ausführungsform weist das akustische Filterelement mehrere hintereinander in den Strömungskanal einmündende Hohlraumresonatoren auf. Dadurch lassen sich im Schalldruckverlauf mehrere negative Wertspitzen an unterschiedlichen Frequenzen verwirklichen. Die genaue Position der Wertspitzen im Frequenzverlauf hängt im Wesentlichen von der Länge der Resonatorhälse ab. Die Auswirkungen, die eine bestimmte Form eines Hohlraumresonators bzw. eine bestimmte Anordnung von Hohlraumresonatoren auf den Schalldruckverlauf haben, kann durch entsprechende routinemäßige Versuche ermittelt werden. Dem Fachmann ist es dadurch möglich, bei Kenntnis der Lehren der gegenständlichen Offenbarung für eine gegebene Messvorrichtung geeignete akustische Filterelemente zu schaffen.

In einer weiteren vorteilhaften Ausführungsform ist zumindest ein akustisches Filterelement auf eine negative Schalldruckverstärkung in zumindest einem zweiten Frequenzbereich, der ein Vielfaches der Grundfrequenz der Nutzfrequenz umfasst, ausgelegt. Durch Kombination mehrerer Hohlraumresonatoren können somit ein oder mehrere Frequenzbereiche effektiv gedämpft und unterdrückt werden.

In einer bevorzugten Ausführungsform ist zumindest ein Hohlraumresonator zylinderförmig ausgebildet. Ein zylinderförmiger Hohlraumresonator ist einfach herzustellen und kann auch einfach gereinigt werden. Überdies kann zur Auslegung des akustischen Filterelements mit verhältnismäßig einfachen Berechnungs- und Simulationsmodellen eine hohe Genauigkeit erzielt werden.

In vorteilhafter Weise sind zumindest zwei Hohlraumresonatoren zylinderförmig ausgebildet und weisen eine unterschiedliche Länge und/oder einen unterschiedlichen Durchmesser auf. Durch Verwendung mehrerer Hohlraumresonatoren mit leicht variierter Länge lassen sich beispielsweise breitere, allerdings nicht so tiefe Einbuchtungen bzw. negative Wertspitzen im Schalldruckverlauf erzielen.

Eine weitere vorteilhafte Ausführungsform sieht vor, dass die Länge zumindest eines Hohlraumresonators verstellbar ist. Dadurch lassen sich die Eigenschaften des akustischen Filterelements beispielsweise an geänderte Umgebungsbedingungen (z.B. einen geänderten Druck, eine andere Temperatur oder ein anderes Messgas) anpassen. Die Längenverstellung kann beispielsweise durch entsprechende Schraubenverbindungen auf einfache Weise realisiert werden.

In einer weiteren vorteilhaften Ausführungsform ist die Messzelle in einem luftdichten Gehäuse angeordnet. Luftdicht bedeutet hier, dass das Gehäuse im Wesentlichen druckdicht ausgeführt und auf Unterdruck und/oder Vakuum bringbar ist. Dazu kann beispielsweise eine Vakuumpumpe an der Messvorrichtung vorgesehen sein, mit der das Gehäuse evakuiert werden kann. Dies dämpft den auf die Messzelle einwirkenden Luftschall und verringert das auf diesem Weg übertragene Störgeräusche. Gemäß technischer Definition wird als Unterdruck ein Druckniveau unterhalb Normaldruck bezeichnet, ein Druckniveau von unter 300 hPa wird als Grob-Vakuum bezeichnet.

In einem weiteren Aspekt sieht das eingangs genannte Verfahren zur Lösung der Aufgabe erfindungsgemäß vor, dass im Strömungskanal zumindest ein akustisches Filterelement vorgesehen wird, das auf eine negative Schalldruckverstärkung in einem ersten Frequenzbereich, der die Grundfrequenz der Nutzfrequenz umfasst, eingestellt wird, wobei das akustische Filterelement an der Zuleitung und/oder der Ableitung angeordnet wird.

In vorteilhafter Weise wird dabei im Strömungskanal zumindest ein akustisches Filterelement vorgesehen, das auf eine negative Schalldruckverstärkung in zumindest einem zweiten Frequenzbereich, der ein Vielfaches der Grundfrequenz der Nutzfrequenz umfasst, eingestellt wird.

In einer weiteren vorteilhaften Ausführungsform umfasst das Einstellen des zumindest einen akustischen Filters ein konstruktives Festlegen und/oder Einstellen zumindest eines oder mehrerer der folgenden Merkmale eines Hohlraumresonators: Form, Position, Länge, Durchmesser, Länge eines zylinderförmigen Hohlraumresonators, Durchmesser eines zylinderförmigen Hohlraumresonators. Dadurch lassen sich akustische Filterelemente durch Verwendung von Hohlraumresonatoren mit bekannten Eigenschaften, wie etwa Helmholzresonatoren oder ähnlichem, in zielgerichteter Weise erstellen. Dies erlaubt des Weiteren eine konstruktiv und rechnerisch einfache Umsetzung der im Verfahren verwendeten akustischen Filterelemente.

Eine weitere bevorzugte Ausführungsform des erfindungsgemäßen Verfahrens sieht vor, dass in einem auf Unterdruck und/oder Vakuum bringbaren Gehäuse, in dem die Messzelle angeordnet ist, ein Unterdruck und/oder Vakuum erzeugt wird.

Die gegenständliche Erfindung wird nachfolgend unter Bezugnahme auf die Figuren 1 bis 10 näher erläutert, die beispielhaft, schematisch und nicht einschränkend vorteilhafte Ausgestaltungen der Erfindung zeigen. Dabei zeigt
Fig.1 eine schematische Darstellung einer erfindungsgemäßen Messvorrichtung,
Fig. 2 ein beispielhaftes Frequenzspektrum eines in einer Messzelle aufgezeichneten Schallsignals,
Fig. 3 eine beispielhafte Darstellung eines akustischen Filterelements mit einem einzigen Hohlraumresonator,
Fig. 4 eine Darstellung des für das akustische Filterelement der Fig. 3 ermittelten Schalldruckverlaufs,
Fig. 5 eine beispielhafte Darstellung eines akustischen Filterelements mit drei hintereinander angeordneten einseitigen Hohlraumresonatoren gleicher Länge,
Fig. 6 eine Darstellung des für das akustische Filterelement der Fig. 5 ermittelten Schalldruckverlaufs,
Fig. 7 eine beispielhafte Darstellung eines akustischen Filterelements mit drei hintereinander angeordneten beidseitigen Hohlraumresonatoren gleicher Länge,
Fig. 8 eine Darstellung des für das akustische Filterelement der Fig. 7 ermittelten Schalldruckverlaufs,
Fig. 9 eine beispielhafte Darstellung eines akustischen Filterelements mit drei hintereinander angeordneten einseitigen Hohlraumresonatoren unterschiedlicher Länge,
Fig. 10 eine Darstellung des für das akustische Filterelement der Fig. 9 ermittelten Schalldruckverlaufs,
Fig. 11 eine beispielhafte Darstellung eines akustischen Filterelements mit drei hintereinander angeordneten beidseitigen Hohlraumresonatoren unterschiedlicher Länge, und
Fig. 12 eine Darstellung des für das akustische Filterelement der Fig. 11 ermittelten Schalldruckverlaufs.

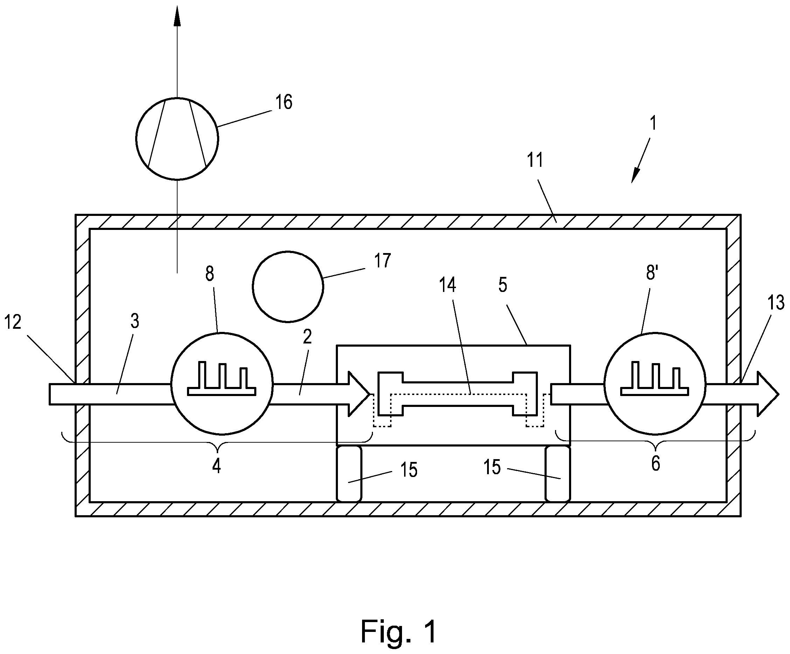

In Fig. 1 sind die grundlegenden Elemente einer Messvorrichtung 1 schematisch dargestellt. Die wesentlichen Bauteile der Messvorrichtung 1 sind in einem Gehäuse 11 geschützt angeordnet, wobei ein Messgas 2 der Messvorrichtung 1 bei einem Messgaseingang 12 zugeführt wird und das Messgas 2 nach der Messung die Messvorrichtung 1 bei einem Messgasausgang 13 wieder verlässt. Der Weg des Messgases 2 im Inneren der Messvorrichtung 1 wird durch einen Strömungskanal 3 definiert, der von dem Messgaseingang 12 zu dem Messgasausgang verläuft. Der Strömungskanal 3 leitet das Messgas 2 über eine photoakustische Messzelle 5, in der das Messgas in an sich bekannter Weise mit einer gepulsten bzw. modulierten Laserstrahlung angeregt wird.

Der Strömungskanal 3 lässt sich somit in drei Abschnitte einteilen: eine Zuleitung 4 vom Messgaseingang 12 bis zur Messzelle 5, einem Strömungsweg 14, der durch die Messzelle führt und dessen Verlauf von dieser definiert ist, und einer Ableitung 6, die von der Messzelle 5 zum Messgasausgang 13 verläuft.

Um die Übertragung von Körperschall von dem Gehäuse 11 auf die Messzelle 5 zu reduzieren, ist die Messzelle 5 auf Dämpfungselementen 15 (z.B. Gummipuffern) gelagert.

Die Dämpfungselemente 15 dämpfen Vibrationen und Umgebungslärm, die von außen auf das Gehäuse 11 einwirken. Um auch den Luftschall, der auf die Messzelle 5 wirkt, zu verringern, kann mit einer Vakuumpumpe 16 im Innenraum des Gehäuses 11 ein Unterdruck oder Vakuum erzeugt werden. Je höher die Qualität des Vakuums ist, desto wirksamer kann Luftschall unterdrückt werden.

Alternativ oder zusätzlich können im Innenraum auch zusätzliche akustische Elemente vorgesehen sein, wie etwa ein Helmholzresonator 17 (gegebenenfalls auch mehrere davon), mit dem eine Verringerung des Geräuschpegels in einem definierten Frequenzbereich erzielt werden kann. Der (die) Helmholzresonator(en) 17 ist (sind) vorzugsweise so abgestimmt, dass Störfrequenzen bei und in der Nähe der Nutzfrequenz(en) der photoakustischen Messzelle 5 absorbiert werden.

Um eine Schallübertragung auf Körperschallweg über die Leitungselemente des Strömungskanals 3 zu verringern, kann dieser zumindest abschnittsweise flexible Schläuche (z.B. aus den Materialien Viton® Tygon®, Silikon oder ähnlichem) aufweisen. Eine Übertragung akustischer Störungen auf Körperschallweg im verwendeten, hörbaren akustischen Frequenzbereich (<10kHz) kann damit weitgehend ausgeschlossen werden.

Lediglich Störungen, die auf dem Luftschallweg über die Zuleitung 4 und die Ableitung 6 zur Messzelle 5 gelangen, können mit den zuvor beschriebenen Maßnahmen kaum wirksam bekämpft werden und stellen daher ein großes Problem dar. Bei diesen Störungen handelt es sich oft um breitbandige Störungen die zum Teil auch die Nutzfrequenz(en) beinhalten und damit zum Messwertrauschen beitragen. Als Quellen dieser Störungen ist neben dem Motorgeräusch selbst (übertragen durch das Abgas- und Entnahmesystem) vor allem das Strömungsrauschen zu nennen. Dieses entsteht an allen Kanten, Querschnittsänderungen, Strömungshindernissen, etc., also vor allem an Bauteilen wie Ventilen, Splittern, Blenden oder Filtern. Je höher der Entnahmefluss ist desto größer das Strömungsrauschen. Um die Dynamik der Messung zu erhöhen, ist es jedoch wünschenswert, den Entnahmefluss zu erhöhen, was auch die Strömungsgeschwindigkeit im Strömungskanal 3 erhöht.

Insbesondere bei mobilen Messgeräten ist der Störschall, der durch die Schläuche übertragen wird, aufgrund der kompakten Bauweise und des geringeren Gewichts problematisch. Vor allem bei einem großen Fluss (beispielsweise mehr als 6 l/min) durch die Messkammer 5 macht sich der Störschall stark bemerkbar.

In diesem Zusammenhang ist auch zu berücksichtigen, dass die akustischen Eigenschaften des Strömungskanals (sowohl in der Zuleitung 4, auch in der Messzelle 5 und in der Ableitung 6) nicht beliebig veränderbar sind, da man dabei auch etwaige negative Effekte berücksichtigen muss. Um etwa die Gaswechselzeiten (Anstiegszeit/Abfallzeit) eines Verbrennungsmotors im Messergebnis berücksichtigen zu können, ist beispielsweise eine möglichst schlanke Form des Strömungskanals 3 bevorzugt, die vor allem im Bereich der Zuleitung 4 und/oder der Ableitung 6 möglichst konstant und insbesondere frei von Puffervolumen bzw. Erweiterungen des Strömungskanals 3 ist. Mit anderen Worten weist der Strömungskanal 3 im Bereich der Zuleitung 4 und/oder der Ableitung 6 einen konstanten Querschnitt auf und ist frei von Puffervolumen. Unter "Bereich" ist hier insbesondere jeweils ein Abschnitt zu verstehen, der einem gewissen Vielfachen des Querschnitts des Strömungskanals 3 entspricht. Hinsichtlich der Zuleitung 4 wäre das beispielsweise zumindest das Dreifache des Querschnitts des Strömungskanals 3 am Messgaseingang 12, hinsichtlich der Ableitung 6 beispielsweise zumindest das Dreifache des Querschnitts des Strömungskanals 3 am Messgasausgang 13. Insbesondere müssen unzulässige Querschnittsveränderungen in der Zuleitung 4 (gegebenenfalls auch in der Ableitung 6) vermieden werden. Als "unzulässig" kann eine Querschnittsveränderung insbesondere dann angesehen werden, wenn sie den zeitlichen Verlauf des Volumenstroms im Bereich der Messzelle 5 gegenüber dem ursprünglich am Messeingang 12 vorherrschenden Verlauf so stark verändert, dass in bestimmten Zeitpunkten die zulässige Messtoleranz überschritten bzw. unterschritten würde.

Um die Auswirkungen der auf dem Luftschallweg über den Strömungskanal 3 zur Messzelle 5 gelangenden Strömungen zu minimieren, ist in der Zuleitung 4 ein akustisches Filterelement 8 vorgesehen, das auf eine negative Schalldruckverstärkung in zumindest einem auf die Nutzfrequenz abgestimmten Frequenzbereich ausgelegt ist. Das akustische Filterelement 8 umfasst zumindest einen, vorzugsweise mehrere an dem Strömungskanal 3 angeordnete Hohlraumresonatoren 7, von denen bevorzugte Ausführungsformen weiter unten beispielhaft beschrieben sind. Die Ausführung des Hohlraumresonators bzw. der Hohlraumresonatoren ist so gewählt, dass diese gesamtheitlich einen akustischen Bandsperr- oder Kerbfilter darstellen. Die Eigenschaften des von dem/den Hohlraumresonatoren 7 definierten akustischen Filterelements kann durch Variation der Form und Abmessung der Hohlraumresonatoren 7, insbesondere deren Längen I und Durchmesser d abgestimmt werden.

Alternativ oder zusätzlich kann ein zweites akustisches Filterelement 8' im Bereich der Ableitung 6 im Strömungskanal 3 angeordnet sein. Das zweite akustische Filterelement 8' kann hinsichtlich seiner Abmessungen und Eigenschaften mit dem ersten akustischen Filterelement 8 übereinstimmen, er kann jedoch auch unterschiedlich gestaltet sein, wenn dies etwa aufgrund unterschiedlicher Abmessungen oder Leitungsdurchmesser sinnvoll erscheint.

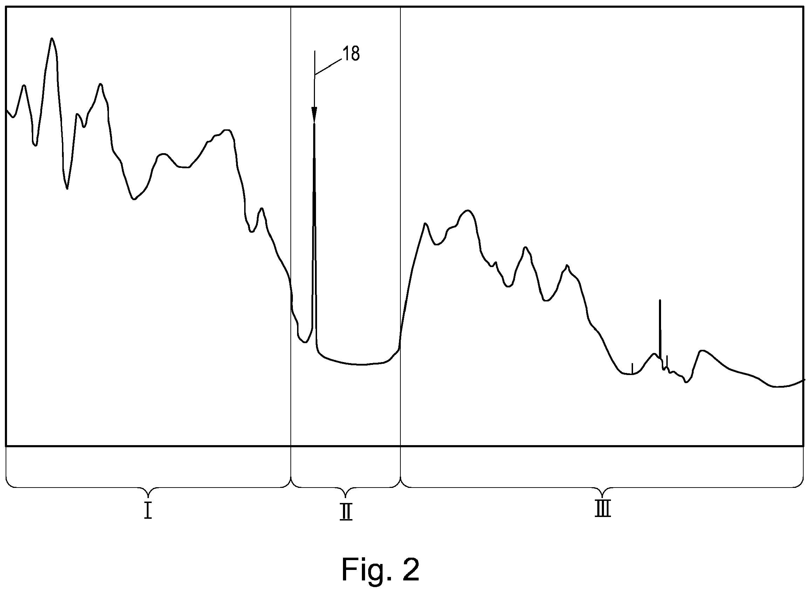

Das gewünschte Verhalten des akustischen Filterelements 8 (und des zweiten akustischen Filterelements 8') wird im Folgenden im Zusammenhang mit Fig. 2 beschrieben. Fig. 2 zeigt ein Frequenzspektrum eines von einer Messzelle 5 aufgezeichneten Schallsignals. Die Messzelle 5 wird dabei mit einer Nutzfrequenz 18 angeregt, wobei die Intensität des Signals bei dieser Nutzfrequenz 18 zur Ermittlung der Messgröße des Messgases 2 ausgewertet wird. Das Frequenzspektrum lässt sich im Wesentlichen in drei Bandbereiche einteilen, einen unteren Bandbereich I der Frequenzen bis 3900 Hz, einen mittleren Bandbereich II der Frequenzen zwischen 3900 Hz und 5100 Hz, und einen oberen Bandbereich III der Frequenzen oberhalb von 5100 Hz. Der mittlere Bandbereich II umfasst die Nutzfrequenz 18, die in diesem Beispiel bei ca. 4100 Hz liegt.

Um den störenden Einfluss des Rauschens im mittleren Bandbereich II zu verringern, wird als akustisches Filterelement 8 einen Bandsperrfilter verwendet, der die Frequenzen im mittleren Bandbereich II dämpft, wie dies deutlich am Verlauf der Frequenzlinie erkennbar ist.

Als akustisches Filterelement 8, 8' wird bevorzugter Weise eine verhältnismäßig einfache Anordnung von zylindrischen Hohlraumresonatoren 7 verwendet, die quer zur Strömungsrichtung in den Strömungskanal 3 einmünden.

Die Anmelder der gegenständlichen Erfindung haben die Wirkung solcher zylindrischer Hohlraumresonatoren 7 auf das frequenzbezogene Schaldruckniveau untersucht. Ziel war es, ein akustisches Filterelement 8 so anzupassen, dass im relevanten Messbereich um die Nutzfrequenz 18 der Rauschpegel gedämpft wird und bei großem und kleinem Durchfluss möglichst gering ist. In diesem Zusammenhang wurden 3D-Simulartionen mit der Simulationssoftware COMSOL durchgeführt, um das grundlegende Verhalten solcher akustischen Filterelemente 8 besser zu verstehen und Verfahren für die Auslegung solcher akustischer Filterelemente 8 zu entwickeln.

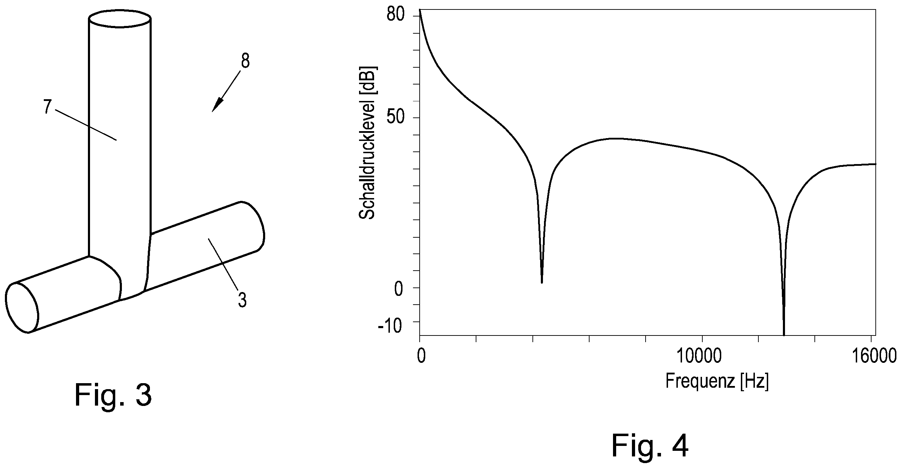

In Fig. 3 bis 12 sind die Simulationsergebnisse jeweils mit einer Darstellung des verwendeten akustischen Filterelements und der damit berechneten Schalldruckverteilung dargestellt.

Die Simulationsreihe wurde anhand mehrerer Modelle durchgeführt, die jeweils eine spezifische Geometrie der Anordnung definieren. Es wurden jeweils nur zylinderförmige Hohlraumresonatoren verwendet. Die Berechnung des Schalldruckverlaufs und weiterer akustischer Größen wurde über einen Frequenzbereich von 20Hz bis 16000Hz durchgeführt und der mit dem Schalldruck logarithmisch in Verbindung stehende Schalldruckpegel geplottet.

Das erste untersuchte akustische Filterelement 8 ist in Fig. 3 dargestellt, der entsprechende Schalldruckverlauf ist in Fig. 4 dargestellt. Der Durchmesser des Strömungskanals 3 beträgt konstant 4mm. Der senkrecht auf den Strömungskanal 3 von diesem abstehende zylindrische Hohlraumresonator 7 hat einen Durchmesser von 5 mm, sowie eine Länge von 21 mm (gemessen von der Achse des Strömungskanals 3 aus). Mit dieser Konfiguration wurde ein akustisches Filterelement mit zwei kerbförmigen negativen Wertspitzen der Schalldruckverstärkung erzielt, wobei die Wertspitzen bei ca. 4100 Hz und bei 12900 Hz liegen.

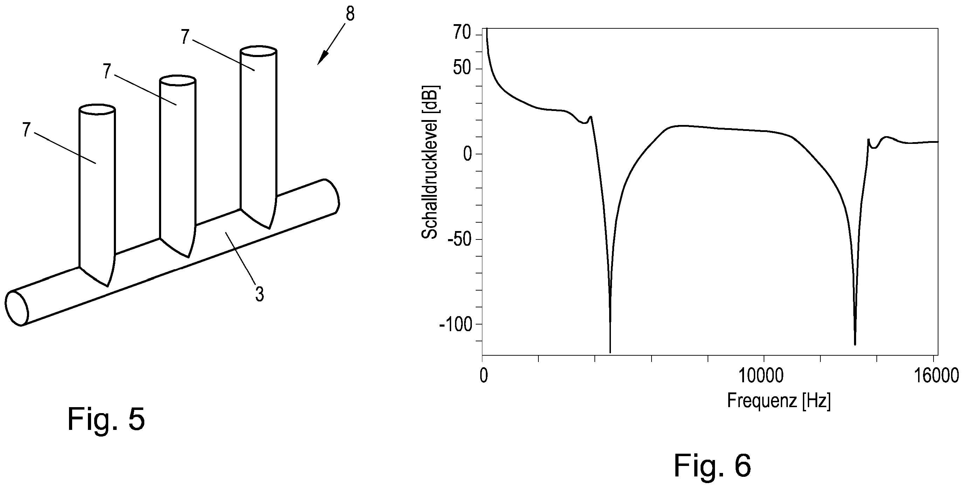

Das zweite untersuchte akustische Filterelement 8 ist in Fig. 5 dargestellt, Fig. 6 zeigt den entsprechend ermittelten Schalldruckverlauf. Der Durchmesser des Strömungskanals 3 beträgt konstant 4mm. Das akustische Filterelement 8 der Fig. 5 weist drei gleiche, einseitig am Strömungskanal 3 angeordnete zylindrische Hohlraumresonatoren 7 mit einer Länge von jeweils 21mm auf. Der Abstand zwischen zwei nacheinander angeordneten Hohlraumresonatoren 7 beträgt 10,5 mm und der Durchmesser jeweils 4mm. Der Schalldruckverlauf dieser Konfiguration weist zwei negative Wertspitzen auf, die im Wesentlichen an der gleichen Stelle angeordnet sind, wie beim ersten Filter der Fig. 3, allerdings sind die Wertspitzen erkennbar breiter und die Dämpfung ist deutlich stärker ausgeprägt.

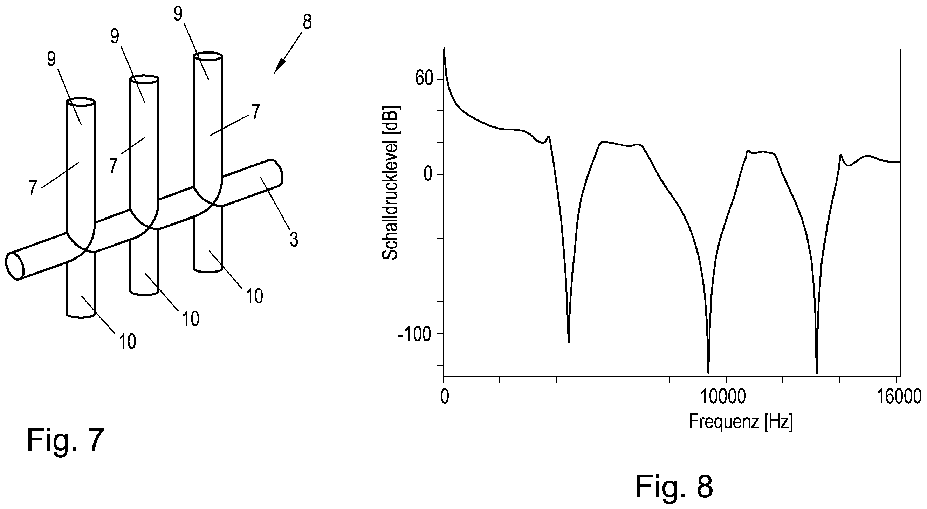

Das dritte untersuchte akustische Filterelement 8 ist in Fig. 7 dargestellt, Fig. 8 zeigt den entsprechend ermittelten Schalldruckverlauf. Der Durchmesser des Strömungskanals 3 beträgt konstant 4mm. Das akustische Filterelement 8 der Fig. 7 weist drei gleiche, am Strömungskanal 3 angeordnete zylindrische Hohlraumresonatoren 7 auf. Die Holraumresonatoren erstrechen sich beidseitig des Strömungskanals 3, wobei einem längeren ersten Hals bzw. Halsabschnitt 9 ein kürzerer zweiter Hals bzw. Halsabschnitt 10 gegenübersteht. Die Länge des ersten Halses 9 beträgt jeweils 21 mm, die Länge des zweiten Halses 10 beträgt jeweils 10,5 mm. Der Abstand zwischen zwei nacheinander angeordneten Hohlraumresonatoren 7 beträgt 10,5 mm und der Durchmesser jeweils 4mm. Verglichen mit dem in Fig. 6 dargestellten Schalldruckverlauf des zweiten untersuchten akustischen Filterelements 8 weist der Schalldruckverlauf dieser Konfiguration eine zusätzliche negative Wertspitzen auf, die zwischen den beiden (weiterhin vorhandenen) bisherigen Wertspitzen bei etwa 9200 Hz liegt. Die mittlere Wertspitze ist dabei deutlich breiter ausgebildet als die beiden seitlichen Wertspitzen.

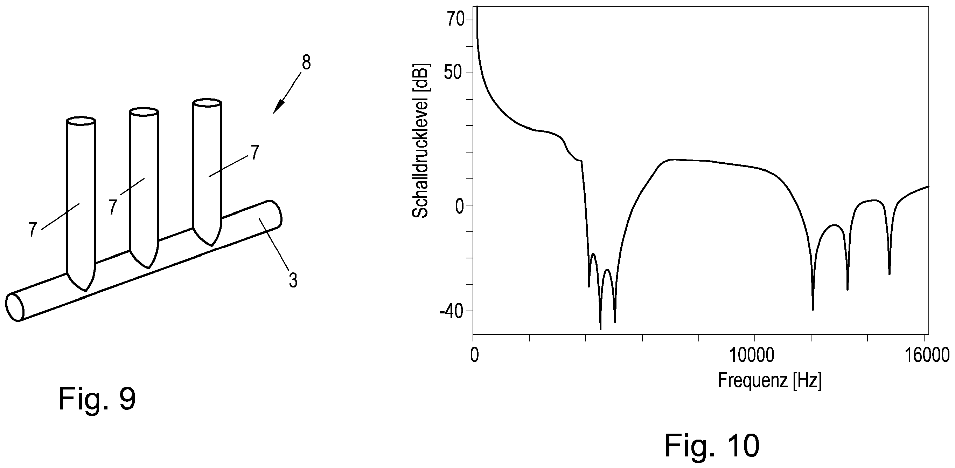

Das vierte untersuchte akustische Filterelement 8 ist in Fig. 9 dargestellt, Fig. 10 zeigt den entsprechend ermittelten Schalldruckverlauf. Der Durchmesser des Strömungskanals 3 beträgt konstant 4mm. Das akustische Filterelement 8 der Fig. 9 weist drei einseitig am Strömungskanal 3 angeordnete zylindrische Hohlraumresonatoren 7 mit jeweils geringfügig voneinander abweichender Länge von 23 mm, 21 mm und 19 mm auf. Der Abstand zwischen zwei nacheinander angeordneten Hohlraumresonatoren 7 beträgt 10,5 mm und der Durchmesser jeweils 4mm. Der Schalldruckverlauf dieser Konfiguration weist zwei ausgeprägte negative Bandbereiche auf, die sich zwischen ca. 4000 Hz und ca. 5000 Hz, bzw. zwischen ca. 11900 Hz und ca. 15000 Hz erstrecken.

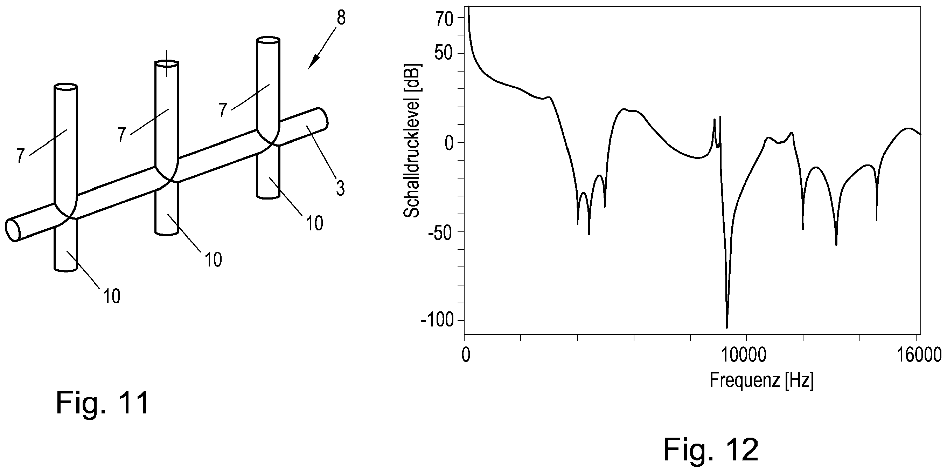

Das fünfte untersuchte akustische Filterelement 8 ist in Fig. 11 dargestellt, Fig. 12 zeigt den entsprechend ermittelten Schalldruckverlauf. Gegenüber dem in Fig. 9 dargestellten Filterelement wurde einerseits der Abstand zwischen den Hohlraumresonatoren 7 auf jeweils 21 mm erhöht und es wurde jeder Hohlraumresonator 7 mit einem zweiten, gegenüberliegenden Hals bzw. Halsabschnitt 10 ergänzt. Die Länge der zweiten Hälse 10 beträgt jeweils 10,5 mm. Die anderen Parameter blieben unverändert. Auch diese Konfiguration schafft zwei ausgeprägte negative Bandbereiche, die sich im Wesentlichen an derselben Stelle befinden, wie schon beim vorherigen Beispiel in Fig. 10. Zusätzlich befindet sich eine sehr ausgeprägte Wertspitze bei ca. 9200 Hz.

Zusätzlich zu der oben dargelegte 3D-Simulation wurde eine Berechnung anhand eines stark vereinfachten zweidimensionalen Modells wiederholt und die Ergebnisse miteinander verglichen. Es zeigte sich, dass die Ergebnisse der 3D-Simulation und der 2D-Berechnung qualitativ bis auf einen Skalierungsfaktor im Wesentlichen äquivalent sind.

Dies verdeutlicht einen erheblichen Vorteil der erfindungsgemäßen, auf einfachen zylindrischen Hohlraumresonatoren 7 basierenden akustischen Filterelemente 8, da eine Auslegung der Filtereigenschaften in einem ersten Schritt anhand einfacher 2D-Modelle erfolgen kann. Eine aufgefundene optimale Konfiguration kann dann anhand komplexerer Modellierungen (etwa in einer Simulation anhand eines 3D-Modells) überprüft oder gleich in einer praktischen Umsetzung getestet werden. (Ein Messergebnis einer solchen praktischen Umsetzung ist beispielhaft in Fig. 2 dargestellt und wurde in Zusammenhang mit der Beschreibung dieser Figur erörtert).

Um dem Fachmann eine Umsetzung der Erfindung in die Praxis zu ermöglichen, wurden von den Erfindern die folgenden Schlussfolgerungen aus den Simulationen und Berechnungen gezogen:
- Die genaue Position der Wertspitzen hängt im Wesentlichen von der Länge der Resonatorhälse ab.
- Die Tiefe (bzw. Höhe) der Wertspitzen hängt von der Anzahl der Resonatoren ab.
- Breitere, allerdings nicht so tiefe Einbuchtungen erreicht man durch die Verwendung mehrerer Resonatorhälsen mit leicht variierender Länge.
- Zusätzliche leichte Verschiebungen können durch Änderungen der Radien und Durchmesser, sowie einer Veränderung der Abstände zwischen den Hälsen erzielt werden, diese Parameter wirken sich jedoch weniger stark aus.

### Bezugszeichen:

Messvorrichtung 1
Messgases 2
Strömungskanal 3
Zuleitung 4
Messzelle 5
Ableitung 6
Hohlraumresonator 7
akustisches Filterelement 8
erster Halsabschnitt 9
zweiter Halsabschnitt 10
Gehäuse 11
Messgaseingang 12
Messgasausgang 13
Strömungsweg 14
Dämpfungselemente 15
Vakuumpumpe 16
Helmholzresonator 17
Nutzfrequenz 18

## Patentansprüche

1. Messvorrichtung (1) zur Ermittlung einer Messgröße eines Messgases (2) mittels eines photoakustischen Verfahrens, wobei die Messvorrichtung (1) einen Strömungskanal (3) für das Messgas (2) mit zumindest einer Zuleitung (4), einer photoakustischen Messzelle (5) und einer Ableitung (6) aufweist, **dadurch gekennzeichnet, dass** die Messvorrichtung (1) zumindest ein auf die Nutzfrequenz (18) der Messzelle (5) abgestimmtes akustisches Filterelement (8) aufweist, das zumindest einen am Strömungskanal (3) angeordneten Hohlraumresonator (7) umfasst, welcher an der Zuleitung (4) und/oder der Ableitung (6) angeordnet ist und dass zumindest eines der akustischen Filterelemente (8) auf eine negative Schalldruckverstärkung in einem ersten Frequenzbereich, der die Grundfrequenz der Nutzfrequenz (18) umfasst, ausgelegt ist.

2. Messvorrichtung (1) nach Anspruch 1, **dadurch gekennzeichnet, dass** die Achse des Hohlraumresonators (7) normal auf die Achse des Strömungskanals (3) ausgerichtet ist.

3. Messvorrichtung (1) nach Anspruch 1 oder 2, **dadurch gekennzeichnet, dass** zumindest ein Hohlraumresonator (7) als beidseitiger Hohlraumresonator (7) mit einem ersten (9) und einem zweiten Halsabschnitt (10) ausgebildet ist, wobei vorzugsweise der erste Halsabschnitt (9) gegenüberliegend dem zweiten Halsabschnitt (10) angeordnet ist.

4. Messvorrichtung (1) nach einem der Ansprüche 1 bis 3, **dadurch gekennzeichnet, dass** das akustische Filterelement mehrere hintereinander in den Strömungskanal (3) einmündende Hohlraumresonatoren (7) aufweist.

5. Messvorrichtung (1) nach einem der Ansprüche 1 bis 4, **dadurch gekennzeichnet, dass** zumindest ein akustisches Filterelement (8) auf eine negative Schalldruckverstärkung in zumindest einem zweiten Frequenzbereich, der ein Vielfaches der Grundfrequenz der Nutzfrequenz (18) umfasst, ausgelegt ist.

6. Messvorrichtung (1) nach einem der Ansprüche 1 bis 5, **dadurch gekennzeichnet, dass** zumindest ein Hohlraumresonator (7) zylinderförmig ausgebildet ist.

7. Messvorrichtung (1) nach Anspruch 6, **dadurch gekennzeichnet, dass** zumindest zwei Hohlraumresonatoren (7) zylinderförmig ausgebildet sind und eine unterschiedliche Länge und/oder einen unterschiedlichen Durchmesser aufweisen.

8. Messvorrichtung (1) nach Anspruch 6 oder 7, **dadurch gekennzeichnet, dass** die Länge zumindest eines Hohlraumresonators (7) verstellbar ist.

9. Messvorrichtung (1) nach einem der Ansprüche 1 bis 8, **dadurch gekennzeichnet, dass** die Messzelle (5) in einem druckdichten, auf Unterdruck und/oder Vakuum bringbaren Gehäuse (11) angeordnet ist.

10. Verfahren zur Unterdrückung von Schallstörungen in einer Messvorrichtung (1) zur Ermittlung einer Messgröße eines Messgases (2) mittels eines photoakustischen Verfahrens, wobei das Messgas (2) über einen Strömungskanal (3) geleitet wird, der über zumindest eine Zuleitung (4), eine photoakustische Messzelle (5) und eine Ableitung (6) verläuft, **dadurch gekennzeichnet, dass** im Strömungskanal (3) zumindest ein akustisches Filterelement (8) vorgesehen wird, das auf eine negative Schalldruckverstärkung in einem ersten Frequenzbereich, der die Grundfrequenz der Nutzfrequenz (18) umfasst, eingestellt wird wobei das akustische Filterelement (8) an der Zuleitung (4) und/oder der Ableitung (6) angeordnet wird.

11. Verfahren nach Anspruch 10, **dadurch gekennzeichnet, dass** im Strömungskanal (3) zumindest ein akustisches Filterelement (8) vorgesehen wird, das auf eine negative Schalldruckverstärkung in zumindest einem zweiten Frequenzbereich, der ein Vielfaches der Grundfrequenz der Nutzfrequenz (18) umfasst, eingestellt wird.

12. Verfahren nach Anspruch 10 oder 11, **dadurch gekennzeichnet, dass** das Einstellen des zumindest einen akustischen Filterelements (8) ein konstruktives Festlegen und/oder Einstellen zumindest eines oder mehrerer der folgenden Merkmale eines Hohlraumresonators (7) umfasst: Form, Position, Länge, Durchmesser, Länge eines zylinderförmigen Hohlraumresonators (7), Durchmesser eines zylinderförmigen Hohlraumresonators.

13. Verfahren nach einem der Ansprüche 10 bis 12, **dadurch gekennzeichnet, dass** in einem auf Unterdruck und/oder Vakuum bringbaren Gehäuse (11), in dem die Messzelle (5) angeordnet ist, ein Unterdruck und/oder Vakuum erzeugt wird.

## Claims

1. Measuring device (1) for determining a measured variable of a measuring gas (2) by means of a photoacoustic method, wherein the measuring device (1) has a flow channel (3) for the measuring gas (2) having at least one feed line (4), a photoacoustic measuring cell (5) and a discharge line (6), **characterized in that** the measuring device (1) has at least one acoustic filter member (8) which is tuned to the useful frequency (18) of the measuring cell (5), wherein the filter member (8) comprises at least one cavity resonator (7) arranged on the flow channel (3) at the feed line (4) and/or at the discharge line (6) and that at least one of the acoustic filter members (8) is designed for a negative sound pressure amplification in a first frequency range which comprises the fundamental frequency of the useful frequency (18).

2. Measuring device (1) according to claim 1, **characterized in that** the axis of the cavity resonator (7) is aligned normally to the axis of the flow channel (3).

3. Measuring device (1) according to claim 1 or 2, **characterized in that** at least one cavity resonator (7) is designed as a cavity resonator (7) having on both sides a first (9) and a second neck portion (10), wherein preferably the first neck portion (9) is arranged opposite the second neck portion (10).

4. Measuring device (1) according to one of claims 1 to 3, **characterized in that** the acoustic filter member has a plurality of cavity resonators (7) entering consecutively into the flow channel (3).

5. Measuring device (1) according to one of claims 1 to 4, **characterized in that** at least one acoustic filter member (8) is designed for negative sound pressure amplification in at least one second frequency range which comprises a multiple of the fundamental frequency of the useful frequency (18).

6. Measuring device (1) according to one of claims 1 to 5, **characterized in that** at least one cavity resonator (7) is formed to be cylindrical.

7. Measuring device (1) according to claim 6, **characterized in that** at least two cavity resonators (7) are formed to be cylindrical and have a different length and/or a different diameter.

8. Measuring device (1) according to claim 6 or 7, **characterized in that** the length of at least one cavity resonator (7) is adjustable.

9. Measuring device (1) according to one of claims 1 to 8, **characterized in that** the measuring cell (5) is arranged in a pressure-tight housing (11) which can be brought to negative pressure and/or vacuum.

10. Method for the suppression of sound interferences in a measuring device (1) for determining a measured variable of a measuring gas (2) by means of a photoacoustic method, wherein the measuring gas (2) is conducted via a flow channel (3) which runs through at least one feed line (4), a photoacoustic measuring cell (5) and a discharge line (6), **characterized in that** at least one acoustic filter member (8) is provided in the flow channel (3), which filter member is adjusted to a negative sound pressure amplification in a first frequency range which comprises the fundamental frequency of the useful frequency (18), wherein the acoustic filter member (8) is arranged at the feed line (4) and/or at the discharge line (6).

11. Method according to claim 10, **characterized in that** at least one acoustic filter member (8) is provided in the flow channel (3), which is adjusted to a negative sound pressure amplification in at least one second frequency range, which comprises a multiple of the fundamental frequency of the useful frequency (18).

12. Method according to claim 10 or 11, **characterized in that** the adjustment of the at least one acoustic filter member (8) comprises a constructive defining and/or adjusting of at least one or a plurality of the following features of a cavity resonator (7): shape, position, length, diameter, length of a cylindrical cavity resonator (7), diameter of a cylindrical cavity resonator.

13. Method according to one of claims 10 to 12, **characterized in that** a negative pressure and/or vacuum is generated in a housing (11) which can be brought to negative pressure and/or vacuum and in which the measuring cell (5) is arranged.

## Revendications

1. Dispositif de mesure (1) permettant de déterminer une grandeur de mesure d'un gaz de mesure (2) au moyen d'un procédé photoacoustique, le dispositif de mesure (1) présentant un canal d'écoulement (3) pour le gaz de mesure (2) comportant au moins une conduite d'alimentation (4), une cellule de mesure (5) photoacoustique et une conduite d'évacuation (6), **caractérisé en ce que** le dispositif de mesure (1) présente au moins un élément filtrant acoustique (8) qui est adapté à la fréquence utile (18) de la cellule de mesure (5) et qui comprend au moins une cavité résonante (7) disposée sur le canal d'écoulement (3), lequel élément filtrant acoustique est disposé sur la conduite d'alimentation (4) et/ou la conduite d'évacuation (6) et **en ce qu'**au moins un des éléments filtrants acoustiques (8) est conçu pour une amplification négative de la pression acoustique dans une première gamme de fréquences, laquelle comprend la fréquence de base de la fréquence utile (18).

2. Dispositif de mesure (1) selon la revendication 1, **caractérisé en ce que** l'axe de la cavité résonante (7) est aligné perpendiculairement à l'axe du canal d'écoulement (3).

3. Dispositif de mesure (1) selon la revendication 1 ou 2, **caractérisé en ce qu'**au moins une cavité résonante (7) est conçue comme une cavité résonante (7) à double face comportant une première (9) et une seconde (10) parties de col, la première partie de col (9) étant de préférence disposée en face de la seconde partie de col (10).

4. Dispositif de mesure (1) selon l'une des revendications 1 à 3, **caractérisé en ce que** l'élément filtrant acoustique présente plusieurs cavités résonantes (7) débouchant de manière successive dans le canal d'écoulement (3).

5. Dispositif de mesure (1) selon l'une des revendications 1 à 4, **caractérisé en ce qu'**au moins un élément filtrant acoustique (8) est conçu pour une amplification négative de la pression acoustique dans au moins une seconde gamme de fréquences qui comprend un multiple de la fréquence de base de la fréquence utile (18).

6. Dispositif de mesure (1) selon l'une des revendications 1 à 5, **caractérisé en ce qu'**au moins une cavité résonante (7) est cylindrique.

7. Dispositif de mesure (1) selon la revendication 6, **caractérisé en ce qu'**au moins deux cavités résonantes (7) sont cylindriques et présentent une longueur différente et/ou un diamètre différent.

8. Dispositif de mesure (1) selon la revendication 6 ou 7, **caractérisé en ce que** la longueur d'au moins une cavité résonante (7) est réglable.

9. Dispositif de mesure (1) selon l'une des revendications 1 à 8, **caractérisé en ce que** la cellule de mesure (5) est disposée dans un boîtier (11) étanche à la pression et qui peut être amené en dépression et/ou sous vide.

10. Procédé permettant de supprimer des interférences sonores dans un appareil de mesure (1) permettant de déterminer une grandeur de mesure d'un gaz de mesure (2) au moyen d'un procédé photoacoustique, le gaz de mesure (2) étant dirigé à travers un canal d'écoulement (3) qui passe par au moins une conduite d'alimentation (4), une cellule de mesure (5) photoacoustique et une conduite d'évacuation (6), **caractérisé en ce qu'**au moins un élément filtrant acoustique (8) est prévu dans le canal d'écoulement (3) et est réglé sur une amplification négative de la pression acoustique dans une première gamme de fréquences, laquelle comprend la fréquence de base de la fréquence utile (18), l'élément filtrant acoustique (8) étant disposé sur la conduite d'alimentation (4) et/ou la conduite d'évacuation (6).

11. Procédé selon la revendication 10, **caractérisé en ce qu'**au moins un élément filtrant acoustique (8) est prévu dans le canal d'écoulement (3) et est réglé sur une amplification négative de la pression acoustique dans au moins une seconde gamme de fréquences, laquelle comprend un multiple de la fréquence de base de la fréquence utile (18).

12. Procédé selon la revendication 10 ou 11, **caractérisé en ce que** le réglage de l'au moins un élément filtrant acoustique (8) comprend une détermination constructive et/ou un réglage constructif d'au moins une ou plusieurs des caractéristiques suivantes d'une cavité résonante (7) : forme, position, longueur, diamètre, longueur d'une cavité résonante (7) cylindrique, diamètre d'une cavité résonante cylindrique.

13. Procédé selon l'une des revendications 10 à 12, **caractérisé en ce qu'**une dépression et/ou un vide sont générés dans un boîtier (11) pouvant être amené en dépression et/ou sous vide et dans lequel est disposée la cellule de mesure (5).
